# EUROPEAN PATENT APPLICATION

(11) **EP 1 164 141 A2**
(43) Date of publication of application: **19.12.2001**
(21) Application number: 01118400.9
(22) Date of filing: 15.02.1995
(51) Int. Cl.: C07K 5/107, A61K 38/07, A61P 25/00

(54) **Opioid peptides and their use for treatment of pain**

(30) Priority: 21.02.1994 GB 9403263; 25.04.1994 GB 9408179; 03.05.1994 SE 9401519
(62) Divisional of application: 95910838.2
(71) Applicant: AstraZeneca AB, 151 85 Södertälje (SE)
(72) Inventor: Brown, William, St-Laurent, Quebec H4S 1Z9 (CA); Dimaio, John, Montreal, Quebec H1E 4L9 (CA); Martel, René, Saint Laurent, Quebec H4L 1Y5 (CA); Schiller, Peter, Montreal, Quebec H3G 2A4 (CA)

(57) **Abstract**

This invention relates to a series of novel opioid peptides for the treatment of pain and pharmaceutically acceptable compositions comprising those peptides. The invention also teaches methods for controlling pain in patients using compositions of the invention. The peptides of this invention have a high degree of selectivity for the µ-opioid receptor. These peptides are highly lipophilic. In spite of their lipophilic character they do not readily cross the blood brain barrier (BBB). The peptides of the present invention are particularly well-suited as analgesic agents acting substantially on peripheral µ-opioid receptors. Because these peptides act peripherally, they substantially avoid producing side effects normally associated with central analgesic action.

## Description

### BACKGROUND OF THE INVENTION

Many endogenous peptides of mammalian and amphibian origin bind to specific opioid receptors and elicit an analgesic response similar to classic narcotic opiates. Many different types of opioid receptors have been shown to coexist in higher animals. For example, see W. Martin et al., J. Pharmacol. Exp. Ther., 197, p. 517(1975); and J. Lord et al., Nature (London), 257, p. 495(1977). Three different types of opioid receptors have been identified. The first, δ, shows a differentiating affinity for enkephalin-like peptides. The second, µ, shows enhanced selectivity for morphine and other polycyclic alkaloids. The third, κ, exhibits equal affinity for either group of the above ligands and preferential affinity for dynorphin. In general, the µreceptors seem to be more involved with analgesic effects. The δ-receptors appear to deal with behavioral effects, although the δ and the κ-receptors may also mediate analgesia.

Each opioid receptor, when coupled with an opiate, causes a specific biological response uniqe to that type of receptor. When an opiate activates more than one receptor, the biological response for each receptor is affected, thereby producing side effects. The less specific and selective an opiate may be, the greater the chance of causing increased side effects by the administration of the opiate.

In the prior art, opiates, opioid peptides, and analogues thereof, have either failed to demonstrate, or have demonstrated a limited degree of specificity and selectivity for the type of receptor, or receptors, to which they bind.

The primary site of action of analgesic opioids is the central nervous system (CNS). Conventional narcotic analgesics are normally quite hydrophobic and thus are extremely well-suited to permeate lipid membranes, such as the blood-brain barrier. Due to this physical capability, analgesics tend to bind with opioid receptors within the central nervous system in the brain. However, they do not necessarily bind with a homogeneous receptor subtype. This binding causes medically undesirable side effects to occur.

Opiates can cause serious and potentially fatal side effects. Side effects such as respiratory depression, tolerance, physical dependence capacity, and precipitated withdrawal syndrome are caused by nonspecific interactions with central nervous system receptors. See K. Budd, In International Encyclopedia of Pharmcology and Therapeutics; N.E. Williams and H. Wilkinson, Eds., Pergammon: (Oxford), 112, p. 51 (1983). Therefore, opioid analgesics acting principally through opioid receptors in the peripheral nervous system would not be expected to cause similar unwanted side effects as those side effects associated with opioid analgesics affecting the central nervous system. The opioid peptides of this invention substantially affect the peripheral nervous system and therefore overcome some of the disadvantages of conventional opiates by substantially preventing the occurence of unwanted side effects.

To date, one of the few classes of agents known to exert peripheral analgesic effects are non-steroidal anti-inflammatory agents, such as aspirin, ibuprofen, and ketorolac. These agents do not interact with opioid receptors but are known to inhibit cyclooxygenase and attenuate prostaglandin synthesis. These weak analgesics do not have centrally mediated side effects, but they can cause other side effects such as ulcerations of the gastro-intestinal tract. It is an object of this invention to provide opioid-like peptides which act peripherally but substantially avoid the unwanted side effects associated with conventional peripherally acting analgesics.

It has recently been shown in the prior art that there is significant peripheral analgesic activity of opiate drugs. See A. Barber and R. Gottschlich, Med. Res. Rev., 12, p.525 (1992) and C. Stein, Anasth. Analg., 76, p.182 (1993). Quaternary salts of known centrally acting opioid alkaloids have been used as pharmacological probes to distinguish between peripheral and central analgesic responses. The quaternary salts of potent opiates have a permanent positive charge and show restricted penetration of the blood-brain barrier. See T.W. Smith et al., Life Sci, 31, p.1205 (1982); T.W. Smith et al., Int. J. Tiss. Reac., 7, p.61 (1985); B.B. Lorenzetti and S.H. Ferreira, Braz. J. Med. Biol. Res., 15, p.285 (1982); D.R. Brown and L.L Goldberg, Neuropharmacol., 24, p.181 (1985); G. Bianchi et al.. Life Sci., 30, p.1875 (1982); and J. Russel et al., Eur. J. Pharmacol., 78, p.255 (1982). Highly polar analogues of enkephalins and dermorphins have been prepared which retain high antinociceptive activity but show limited central nervous system penetration. See R.L. Follenfant et al., Br. J. Pharmacol., 93,p.85 (1988); G.W. Hardy et al., J. Med. Chem., 32, p.1108 (1989). Conversely, in the prior art, lipophilic opioid peptides were thought to more readily penetrate the blood-brain barrier. Surprisingly, the opioid peptides of this invention are highly lipophilic but do not significantly penetrate the blood brain barrier.

Unlike conventional opiates, opioid peptides are hydrophobic. Their hydrophobicity tends to enhance their rate of elimination from the mammalian body. Hydrophobicity increases theses peptide's capacity to traverse epithelial barriers. Notwithstanding, administration of opioid peptides into the mammalian body has been shown to affect the central nervous system. Therfore, much effort has been expended on improving the absorption properties of these compounds. Scientists have attempted to lessen the peptide's penetration of the central nervous system especially if prolonged exposure of the body to these chemicals could cause undesirable side effects or even be toxic.

It was thought that non-poalar peptides pass more easily into the central nervous system than polar peptides by traversing the blood-brain barrier. It has been published that TAPP (H-Tyr-D-Ala-Phe-Phe-NH₂) exhibited antinociceptive properties both peripherally and centrally ( P. Schiller et al., Proceedings of the 20th European Peptide Symposium, 1988). In contradiction, it has been found by the present inventors that this tetrapeptide TAPP (H-Tyr-D-Ala-Phe-Phe-NH₂) does not act centrally. This result was shown by the lack of analgesic effect even at doses of 100mg/kg in the mouse hot plate test This test is standard and known to persons skilled in the art. The test detects chemicals that exert a centrally mediated analgesic response.

The term "specificity" as used in this application refers to the particular or definitive binding of an opiate or opioid peptide to one particular opioid receptor over another opioid receptor. The specificity of an opioid peptide is indicated with the binding inhibition constant, Kᵢ. The term "selectivity" refers to the ability of an opiate or opioid peptide to discriminate among several opioid receptors and to bind to only one particular receptor. The selectivity of an opioid peptide for the µ-receptor is indicated through a ratio of binding inhibition constants. For instance, the ratio of binding inhibition constants, Kᵢ^{δ}/Kᵢ^{µ}, is a value that may be used to measure selectivity. This ratio represents the relationship of the affinities for binding to the µ and δ receptors. A higher value for this ratio indicates a greater preference of ligand to bind with the µ receptor over the δ receptor. One conventional opioid peptide analog, H-Tyr-D-Ala-Gly-Phe(NMe)-Gly-ol (DAGO), is known to be one of the most µ selective opioid peptide analogues. This peptide shows a Kᵢ^{δ}/Kᵢ^{µ} value of 1050. Leu-enkephalin, on the other hand, shows a Kᵢ^{δ}/Kᵢ^{µ} value of 0.2. This fractional value reflects a pronounced affinity for the δ receptor over the µ receptor.

A peptide must have certain attributes to be pharmacologically useful. First, a peptide should be resistant to proteolytic degradation. Second, a peptide should cause an enhanced biological response. Third, a peptide must be safe for human consumption. Fourth, a peptide should be capable of being synthesized in quantities large enough to use in clinical studies respecting its toxicity and later for commercialization. In the present case, less lipid solubility and greater aqueous solubility are also desirable properties for the peptides to possess, to prevent permeation through the blood-brain barrier and to permit rapid excretion of any excess administered peptide and its metabolites. Further, it would be desirable for a peptide to elicit selective and specific receptor binding activity, in order to minimize potential side effects.

There is a need for peptides which act on one specific opioid receptor, specifically the µ receptor. It would be desirable to find peptides with less lipid solubility than that of conventional opiates so that the blood-brain barrier would not be breached. Further, peptides of high polarity would normally be more soluble in aqueous media of physiological pH, thereby enhancing their excretion and the excretion of theif metabolites.

### SUMMARY OF THE INVENTION

The present invention provides for novel compounds which are selective and specific for substantially one opioid receptor. The present invention provides peptides which exhibit a preferential selectivity and specificity for the µ-opioid receptor. The invention also provides for peptides which primarily interact with opioid receptors on peripheral nerve terminals and do not substantially cross the blood-brain barrier. The present invention therefore reduces the severity and number of side effects as compared to the side effects assodated with conventional opiates and opioid peptides reported to date.

The compounds of the present invention are represented by formula (1): 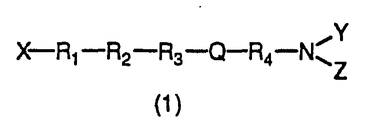 and derivatives and analogues thereof,
wherein
X is selected from the group consisting of H and C₁₋₆ alkyl;
Y and Z are independently selected from the group consisting of H, cyclic aralalkyl, and C₁₋₆ alkyl;
R₁ is a tyrosyl residue, 2', 6'-dimethyltyrosyl residue, or an analog or derivative thereof;
R₃ is an aromatic amino acid;
R₄ is an aromatic amino acid residue;
R₂ is an amino acid having the R-configuration with the provisos that
   when R₁ is a tyrosyl residue, R₂ is D-alanine, X, Y, and Z are H, and R₃ is phenylalanine, then R₄ is not phenylalanine unsubstituted or substituted with 4NO₂ or 4N₃;
   when R₁ is a tyrosyl residue, R₂ is D-alanine, X, Y, and Z are H, and R₄ is phenylalanine, then R₃ is not phenylalanine unsubstituted or substituted with 4NO₂;
   when R₁ is a tyrosyl residue, R₂ is D-alanine, X, Y, and Z are H, and R₄ is 1'-naphthylalanine, then R₃ is not 1'-naphthylalanine or 2'-naphthylalanine; and
   when R₁ is a tyrosyl residue, R₂ is D-alanine, and X, Y, and Z are H, then both R₃ and R₄ are not tryptophan; and
Q is an amide bond or an interposed amide bond mimetic.

The invention also provides for pharmaceutically acceptable compositions comprising those peptides, for use in the treatment of pain.

The invention also provides the use of those peptides for the manufacture of peripheral analgesics for the treatment of pain.

The invention further provides the use of a peptide of formula H-Tyr-D-Ala-Phe-Phe-NH₂ for the manufacture of a peripheral analgesic for the treatment of pain.

### TABLES AND FIGURES

Table 1 lists *in vivo* and *in vitro* activity of hydrophobic dermorphin related tetrapeptides.

Figure 1 indicates the time course of the analgesic effect of morphine (10mg kg⁻¹) (Fig A) and exemplary test compounds (Fig B: BCH2463; C: BCH2462; D: BCH2687)

Figure 2 shows dose response curves for BCH2463 in the phenyl quinone-induced writhing assay (in the mouse s.c.) ED₅₀ = 0.5 mg kg⁻¹ at 20 minutes post administration.

Figure 3 lists comparative analgesic time course of BCH1774 and BCH2463 in the phenyl quinone-induced writhing assay (mouse s.c.)

### DESCRIPTION OF THE INVENTION

The following common abbreviations are used throughout the specification and in the claims:

| | |
|---|---|
| Abu - aminobutyric acid | Aib - aminoisobutyric acid |
| Ala - alanine | Chl - cyclohomoleucine |
| Arg - arginine | Cys (Bzl) - cysteine (benzyl) |
| Cle - cycloleucine | Dmt - 2'6'-dimethyltyrosyl |
| Gin - glutamine | Glu - glutamic acid |
| Gly - glycine | GPI - guinea pig ileum |
| His - histidine | Ile - isoleucine |
| Hph - homophenyl alanine | Met - methionine |
| Leu - leucine | MVD - mouse vas deferens |
| Nle - norleucine | Nva - norvaline |
| Phe - phenylalanine | Pro - proline |
| Phg - phenylglycine | |
| Ser - serine | Thr - threonine |
| Trp - tryptophan | Tyr - tyrosine |
| Nal - 1'-, or 2'-naphthylalanine | |
| PBQ - phenyl-p-benzoquinone | |
| Tic - tetrahydroisoquinoline-3-carboxylic acid | |
| TAPP - H-Tyr-D-Ala-Phe-Phe-NH₂ | |
| TSPP - H-Tyr-D-Ser-Phe-Phe-NH₂ | |

The term "amino acid", and "aromatic amino acid", as used herein, includes naturally occurring amino adds as well as non-natural amino acids, their derivatives, and analogues, commonly utilized by those skilled in the art of chemical synthesis and peptide chemistry. Also analogues of TAPP where the phenyl alanine is para-substituted at position 4 with a nitro or azido residue are included. A list of non-natural and non-proteogenic amino acids may be found in "The Peptide", vol 5, 1983, Academic Press, Chapter 6 by D.C. Roberts and F. Vellaccio which is incorporated herein by reference. Examples of aromatic amino adds include tyrosine, tryptophan, phenylglycine, histidine, naphthylalanine, tetrahydroisoquiniline-3carboxylic add and benzylcysteine. Other examples of aromatic amino adds include phenylalanine substituted on its aromatic ring with, for example, CH₃, C₂H₅, F, Cl, Br, NO₂, OH, SH, CF₃, CN, COOH, and CH₂COOH or substituted at the β-carbon with a lower alkyl radical, OH, SH, or a benzene group. The aromatic ring may be multisubstituted. Aromatic amino adds may also include aromatic carbocycles of the phenylglycine type where the aromatic ring of phenylglycine is substituted with CH₃, C₂H₅, F, Cl, Br, NO₂, OH, SH, CF₃, CN, COOH, and CH₂COOH. These examples are intended to be exemplary only and are not intended to limit the invention in any way.

The term "ED₅₀" as shown in table 1 for the PBQ writhing assays is defined as the dose of drug which induces a 50% reduction in the number of writhes observed compared to the control. The term "ED₅₀" used in the hot-plate assays is defined as the dose of drug required to increase the latency of response 2-fold compared to controls and was determind by parallel-line probit analysis.

The term "interposed amide bond mimetic" is a bond in which the carbonyl group and the NH group of an amide bond are interchanged.

The term "Kᵢ" is the binding inhibition constant. The term "Kᵢ^{δ}/Kᵢ^{µ}" is a value that may be used to measure selectivity. This ratio represents the relationship of the affinities of opioid peptides for binding to the µ- and δ-receptors.

The term "R-configuration" refers to the three dimensional arrangement of substituents around a chiral element. A general system for designating absolute configuration is based upon a priority system which is well-known to persons skilled in the art and is briefly described hereafter. Each group attached to the chiral center is assigned a number according to priority. The molecule is viewed from the side opposite the lowest priority. The configuration is specified "R" if the eye proceeds in a clockwise direction when traveling from the group of highest priority to the group of lowest priority.

The term "residue" when applied to an amino acid, means a radical derived from the corresponding amino acid by removing the hydroxyl of the carboxyl group and one hydrogen from the amino group.

The compounds of the present invention are represented by formula (1): 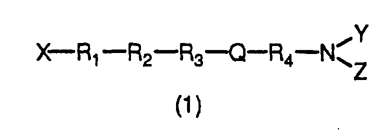 and derivatives, and analogues thereof,
wherein,
X is selected from the group consisting of H and C₁₋₆ alkyl;
Y and Z are independently selected from the group consisting of H, cyclic aralkyl, and C₁₋₆ alkyl;
R₁ is a tyrosyl residue, 2', 6'-dimethyltyrosyl residue, or an analog or derivative thereof;
R₃ is an amino acid residue selected from the group consisting of aromatic amino acids;
R₄ is an aromatic amino acid residue
R₂ is an amino acid having the R-configuration with the proviso that
   when R₁ is a tyrsoyl residue, R₂ is D-alanine, X, Y, and Z are H, and R₃ is phenylalanine, then R₄ is not phenylalanine unsubstituted with 4NO₂ or 4N₃;
   when R₁ is a tyrosyl residue, R₂ is D-alanine, X, Y, and Z are H, and R₄ is phenylalanine, then R₃ is not phenylalanine unsubstituted or substituted with 4NO₂;
   when R₁ is a tyrosyl residue, R₂ is D-alanine, X, Y, and Z are H, and R₄ is 1'-naphthylalanine, then R₃ is not 1'-naphthylalanine or 2'-naphthylalanine; and
   when R₂ is D-alanine, R₁ is a tyrosyl residue, and X, Y, and Z are H, then both R₃ and R₄ are not tryptophan;
   and
Q is an amide bond or an interposed amide bond mimetic.

Preferred compounds are represented by formula (1) and derivatives and analogues thereof,
wherein X is H.

Other preferred compounds are represented by formula (1) and derivatives and analogues thereof,
wherein
R₂ is an amino acid residue having the R-configuration with the proviso that
   where R₁ is a tyrosyl residue, R₂ is a D-alanine, and X, Y, and Z are H, then R₃ and R₄ are different and are selected from the group consisting of phenylalanine and tryptophan.

Other preferred compounds are represented by formula (1) and derivatives and analogues thereof,
wherein
Q is an amide bond or an interposed amide bond mimetic of the formula Q₁-Q₂ wherein Q₁ is selected from the group consisting of CH₂, CHOH, C=O, C=S, and CH=, and Q₂ is selected from the group consisting of CH₂, NH, S, SO, SO₂, O and CH= with the proviso that when Q₁ is CH=, then Q₂ is CH=.

Further preferred compounds are represented by formula (1) and derivatives and analogues thereof,
wherein,
Y and Z are H;
R₃ and R₄ are independently an aromatic amino acid; and R₂ is an amino acid having the R-configuration with the proviso that when R₁ is a tyrosyl residue, and R₂ is D-alanine, then R₃ and R₄ are different and are selected from the group consisting of phenylalanine and tryptophan.

Further preferred compounds are represented by formula (1) and derivatives and analogues thereof,
wherein,
R₂ is an amino acid having the R-configuration with the proviso that R₂ is not D-alanine; and
R₃ and R₄ are phenylalanyl residues.

Still, further preferred compounds are represented by formula (1) and derivatives and analogues thereof,
wherein,
R₁ is a tyrosyl residue;
R₂ is selected from the group consisting of D-norvaline,
D-serine, and D-arginine;
R₃ and R₄ are phenylalanyl residues; and
Q is a peptide bond.

More preferred compounds are represented by formula (1) and derivatives and analogues thereof,
wherein,
X is H,
Y and Z are independently selected from the group consisting of H, aralkyl, and C₁₋₆ alkyl,
R₁ is a tyrosyl residue, 2', 6'-dimethyltyrosyl residue, or an analogue or derivative thereof,
R₃ is an aromatic acid,
R₄ is independently selected from the group consisting of aromatic and aliphatic amino acid, and
R₂ is an amino acid residue having the R-configuration with the proviso that where R₂ is D-alanine, R₁ is a tyrosyl residue, and Y and Z are H, then R₃ and R₄ are independently selected from the group consisting of phenylalanine, and tryptophan, but are not the same, Q is an amide bond or an interposed amide bond mimetic of the formula Q₁-Q₂ wherein Q₁ is selected from the group consisting of CH₂, CHOH, C=O, C=S, and CH=, and Q₂ is selected from the group consisting of CH₂, NH, S, SO, SO₂, O and CH= with the proviso that Q₁ is CH=, then Q₂ is CH=.

More preferred compounds are represented by formula (1) and derivatives and analogues thereof,
wherein,
X is H,
Y and Z are H,
R₁ is a tyrosyl residue, a 2', 6'-dimethyltyrosyl residue, or an analogue or derivative thereof,
R₃ and R₄ are independently an aromatic amino acid,
R₂ is an amino acid having the R-configuration with the proviso that when
R₂ is D-alanine, and R₁ is a tyrosyl residue, then R₃ and R₄ are independently selected from the group consisting of phenylalanine and tryptophan, but are not the same,
Q is an amide bond or an interposed amide bond mimetic of the formula Q₁-Q₂ wherein Q₁ is selected from the group consisting of CH₂, CHOH, C=O, C=S, and CH=, and Q₂ is selected from the group consisting of CH₂, NH, S, SO, SO₂, O, and CH=, with the proviso that when Q₁ is CH=, then Q₂ is CH=.

More preferred compounds are represented by formula (1) and derivatives and analogues thereof,
wherein,
X is H,
Y and Z are H,
R₁ is a tyrosyl residue, 2', 6'-dimethyltyrosyl residue, or an analogue or derivative thereof,
R₂ is an mino acid having the R-configuration with the proviso that R₂ is not alanine,
R₃ and R₄ are phenylalanyl residues,
Q is an amide bond or an amide bond mimetic of the formula Q₁-Q₂
wherein Q₁ is selected from the group consisting of CH₂, CHOH, C=O, C=S, and CH=, and Q₂ is selected from the group consisting of CH₂, NH, S, SO, SO₂, O, and CH=, with the proviso that when Q₁ is CH=, then Q₂ is CH=.

Most preferred compounds are represented by formula (1) and derivatives and analogues thereof,
wherein,
X is H,
Y and Z are H,
R₁ is a tyrosyl residue,
R₂ is selected from the group consisting of D-norvaline, D-serine, and
D-arginine,
R₃ and R₄ are phenylalanyl residues, and
Q is a peptide bond.

Preferred compounds of this invention are listed as follows:
H-Tyr-D-Phe-Phe-Phe-NH₂
H-Tyr-Aib-Phe-Phe-NH₂
H-Tyr-D-Nle-Phe-Phe-NH₂
H-Tyr-Pro-Phe-Phe-NH₂
H-Tyr-D-Ala-Phe-2'-Nal-NH₂
H-Tyr-D-Ala-D-Phe-Phe-NH₂
H-Tyr-D-Ala-Phe(4NO₂)-Phe(4NO₂)-NH₂ H-Tyr-D-Ala-Phe-Tic-NH₂
H-Tyr-D-Ala-Phe-Phe(NMe)-NH₂
H-Tyr-D-Ala-Phe-1'Nal-NH₂
H-Tyr-D-Ala-Trp-Phe-NH₂
H-Tyr-D-Ala-Phe-Trp-NH₂
H-Tyr-VAla-Phe-Phe-NH₂
∇CH₂-Tyr-D-Ala-Phe-Phe-NH₂
H-Tyr-D-Nle-Phe-Trp-NH₂
H-Tyr-D-Nle-Phe-2'-Nal-NH₂
H-Tyr-D-Me-Trp-Phe-NH₂
H-Tyr-D-Ala-Trp-2'-Nal-NH₂
H-Tyr-D-Nle-Trp-2'-Nal-NH₂
H-Tyr-D-Nle-Trp-Trp-NH₂
H-Tyr-D-Nva-Phe-Phe-NH₂
H-Tyr-D-Ser-Phe-Phe-NH₂
H-Tyr-D-Val-Phe-Phe-NH₂
H-Tyr-D-Leu-Phe-Phe-NH₂
H-Tyr-D-Ile-Phe-Phe-NH₂
H-Tyr-D-Abu-Phe-Phe-NH₂
H-Tyr-Chl-Phe-Phe-NH₂
H-Tyr-Cle-Phe-Phe-NH₂
H-Tyr-D-Arg-Phe-Phe-NH₂
H-Tyr-D-Cys-Phe-Phe-NH₂
H-Tyr-D-Thr-Phe-Phe-NH₂
H-DMT-D-Ser-Phe-Phe-NH₂
Tyr-D-Ala-Phe-Phe-OH trifluoroacetate
H-Tyr-D-Ala-Phe-Phg-NH₂ trifluoroacetic acid salt
H-Tyr-D-Arg-Phe-Hph-NH₂ bis-trifluoroacetic acid
H-DMT-D-Ala-Phe-Phe-NH₂ trifluoroacetic acid
H-D-DMT-D-Ala-Phe-Phe-NH₂ trifluoroacetic add salt
H-Tyr-D-Ala-Phe-Hph-NH₂ trifluoroacetic acid salt
H-Tyr-D-Ala-Phe-Cys(Bzl)-NH₂ trifluoroacetic acid salt
H-Tyr-D-Arg-Hph-Phe-NH₂ bis-trifluoroacetic add salt
H-Tyr-D-Arg-Phg-Phe-NH₂ bis-trifluoroacetic acid salt
Tyr-D-Ala-Phe-Phe-CH₂OH hydrochloride salt
H-Tyr-D-Ala-Hph-Phe-NH₂ trifluoroacetic acid salt
H-Tyr-D-Met-Phe-Phe-NH₂ trifluoroacetic acid salt
H-Tyr-D-Arg-Phe-D-Phe-NH₂ bis-trifluoroacetic acid salt
H-Tyr-D-Ala-Phg-Phe-NH₂ trifluoroacetic acid salt
H-Tyr-(D)-Ala-(D)-Phg-Phe-NH₂ trifluoroacetic acid salt
H-Tyr-D-Arg-Phe-Phe(pf)-NH₂ bis-trifluoroacetic acid salt
H-Tyr-D-Arg-Phe-D-Phe(pf)-NH₂ ditrifluoroacetic acid salt
H-Tyr-D-Ala-Phe-Phe(pf)-NH₂ trifluoroacetic acid salt
H-Tyr-D-Ala-Phe-D-Phe(pf)-NH₂ trifluoroacetic add salt

More preferred compounds of this invention are listed as follows:
H-Tyr-D-Nva-Phe-Phe-NH₂
H-Tyr-D-Ser-Phe-Phe-NH₂
H-Tyr-D-Arg-Phe-Phe-NH₂

The best mode of carrying out the invention known at present is to use the compound H-Tyr-D-Arg-Phe-Phe-NH₂

The invention also includes the use of the compound TAPP H-Tyr-D-Ala-Phe-Phe-NH₂ as a peripheral analgesic.

A number of tetrapeptides based on the general formula 1, have been prepared and evaluated as opioid receptor ligands and systemically acting analgesic agents. These compounds are listed in Table 1 along with their respective binding inhibition constants and receptor selectivity ratios.

2', 6'-dimethyltyrosine (Dmt) may be substituted for tyrosine in the opioid peptide compounds. Experiments have shown that the substitution of Dmt for tyrosine at the R₁ position, the first amino acid residue in general formula 1, enhances the potency of the opioid peptide at the µ-receptor up to 2 orders of magnitude. The selectivity for the µ-receptor increases when the compound includes Dmt at the R₁ position. This substitution causes a corresponding shift in the ratio of binding inhibition constants to reflect the increased µ-receptor selectivity.

Many of the compounds listed in Table 1 show good µ-receptor binding but show weak analgesic effect in the mouse writhing assay. This anomaly may be due to rapid proteolysis, rapid clearence, or both. For example, when the prototype lipophilic dermorphin peptide TAPP (BCH1774) was exposed to brushborder kidney membranes, it was observed to be rapidly degraded within 15-30 minutes. Of the peptides listed in Table 1, three preferred compounds other than TAPP itself exhibit an increased analgesic effect *in vivo.* These three compounds are H-Tyr-D-Nva-Phe-Phe-NH₂ (BCH2462), H-Tyr-D-Ser-Phe-Phe-NH₂ (BCH2463), and H-Tyr-D-Arg-Phe-Phe-NH₂ (BCH2687). BCH2462, BCH2463, and BCH2687 have been shown to exhibit peripheral analgesia. No central analgesic effect was observed using these peptides even at doses of 100 mg/kg in the mouse hot plate test.

As shown in Table 1, the ED₅₀ value for TAPP (BCH1774) is 1.4. The corresponding values for H-Tyr-D-Nva-Phe-Phe-NH₂ (BCH2462), and H-Tyr-D-Ser-Phe-Phe-NH₂ (BCH2463), and H-Tyr-D-Arg-Phe-Phe-NH₂ (BCH2687) are 2.7, 0.5, and 0.5 respectively. The ED₅₀ values for the remaining compounds in Table 1 are higher than these figures. Although the ED₅₀ value of BCH2813 was only 0.15, it was found to act centrally at doses of about 40 mg/kg in the hot plate test.

These results indicate that the compounds BCH1774, BCH2462, and BCH2463 still undergo proteolysis but they have a longer half life and therefore are more effective as analgesic agents. In Figure 6, the duration in vivo of analgesic effects caused by BCH1774 (TAPP) and BCH2463 (TSPP) were compared. Using 30 mg/kg s.c. of BCH2463 and 20 mg/kg s.c. of BCH1774, Figure 3 indicates that the analgesic effect of BCH1774 lasted longer than for BCH2463 possibly indicating a slightly accelerated *in vivo* proteolysis of BCH2463 than for BCH1774.

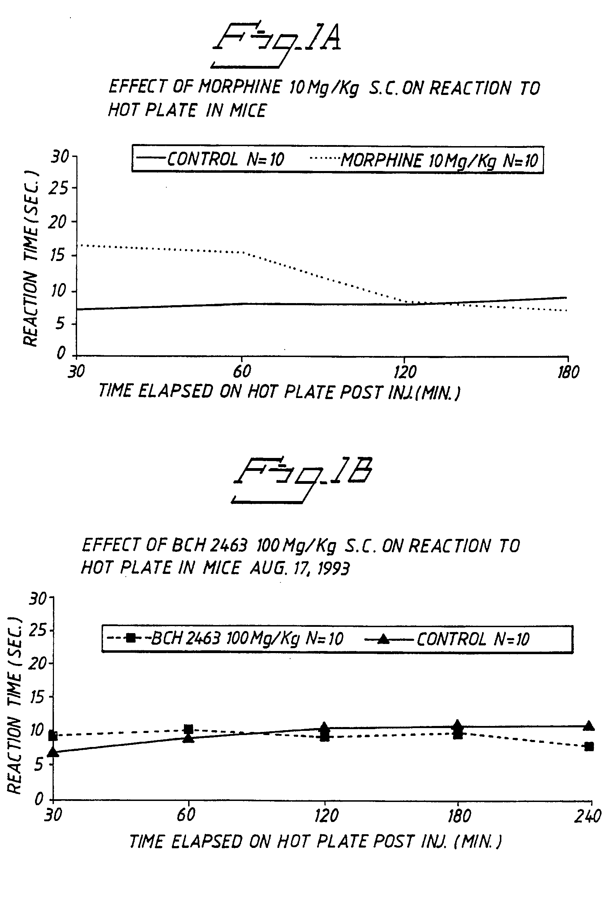

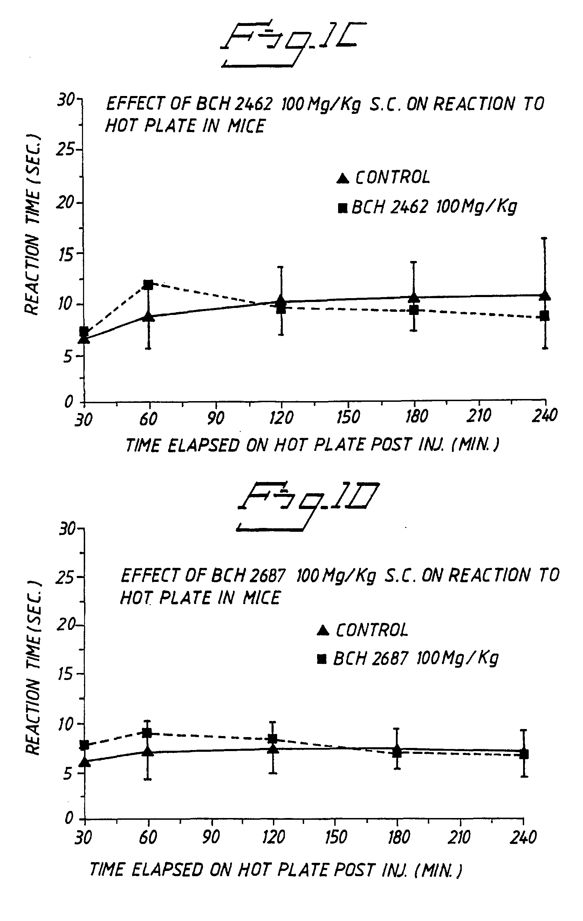

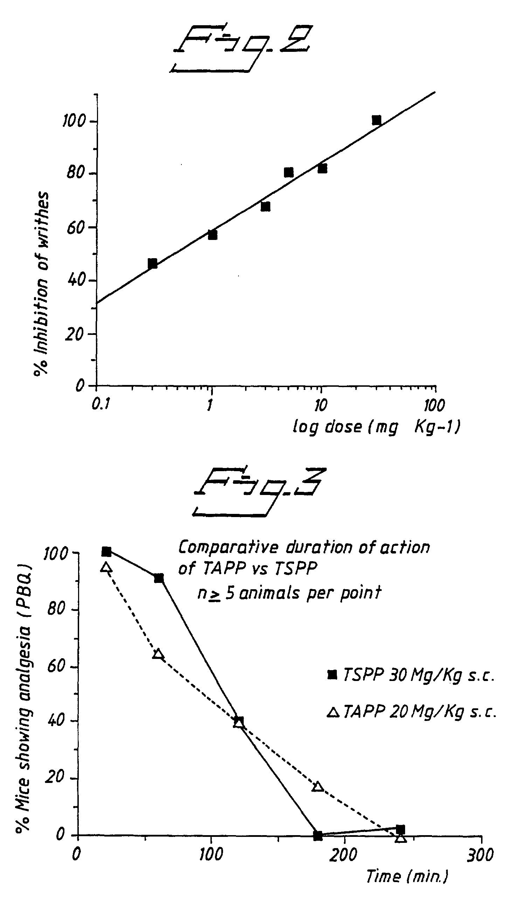

Figures 1A-D show the effects of morphine, BCH2463 (TSPP), BCH2462 (TNPP), and BCH2687 in mice by evaluating the reaction of the mice in the hot plate test. As shown in Figure 1A, the reaction time of the mice treated with 10 mg/kg of morphine is approximately 17 seconds. The reaction time of the mice treated with 100 mg/kg of BCH2463 (Fig 1B) is about 9 seconds compared to a control value of approximately 7 seconds. These results indicate that while morphine inhibits the nociceptive thermal stimulus, BCH2463 does not; but BCH2463 is a potent analgesic agent as is shown by the inhibition of chemically-induced writhing (Fig 2). The reaction time of the mice treated with BCH2462 and with BCH2687 (Figs. 1C, 1D) is approximately 8 seconds which indicates similar results as for BCH2463.

The effects of inhibition of proteolytic metabolism of BCH2463 by the inhibitor DL-Thiorphan has been studied and also the metabolic breakdown of BCH2463 mediated by brush border kidney membranes. The data obtained indicate that the kidney may be the principal site of clearance and metabolism for the compound BCH2463. From Figure 2, it appears that the endopeptidase enzyme EC24-11, which is inhibited by DL-thiorphan, is the preliminary mediator of BCH2463 proteolysis by brush border kidney extract.

Both BCH1774 (TAPP) and BCH2462 (TNPP) exhibited lethal effects upon mice when administered at 1-5 mg kg⁻¹ i.v. bolus dose of drug. In contrast, BCH2463 (TSPP) surprisingly did not exhibit any lethal effects at doses up to 20 mg kg⁻¹ i.v. In addition, peptides were safe when administered subcutaneously (s.c.) at doses greater than 100 mg kg⁻¹. Therefore, the desired route of administration for these compounds is subcutaneous. Thus, the structural paradigm exemplified by BCH1774 can be modified while maintaining exclusion from the central nervous system even at doses as high as 100 mg kg⁻¹ s.c. and the deleterious i.v. toxicity can be minimized. Thus BCH2463 is not lethal to mice at doses at least as high as 20 mg kg⁻¹ (i.v.).

Pharmaceutically acceptable salts of the peptides of this invention may be formed conventionally by reaction with an appropriate acid. Suitable acid addition salts may be formed by the addition of acids such as hydrochloric, hydrobromic, phosphoric, acetic, fumaric, salicyclic, citric, lactic, mandelic, tartaric, oxalic, methanesulphonic, and other suitable adds known to persons skilled in the art.

The present invention also provides for pharmaceutical compositions. Suitable compositions have a pharmaceutically effective amount of the peptide of this invention, or pharmaceutically acceptable salts thereof, and a pharmaceutically acceptable carrier or adjuvant.

The present invention also provides for a method of treatment of pain in animals, such as mammals, including humans. The method comprises the steps of administering a pharmaceutically effective amount of a peptide of formula 1 or a pharmaceutically acceptable salt thereof, to the patient. A pharmaceutical composition as described above may also be used.

The following examples are used to better describe the invention. These examples are for the purpose of illustration only, and are not intended to limit the invention in any manner.

### EXAMPLES

The opioid activity of the peptides was assessed *in vitro* using the guinea pig ileum (GPI) longitudinal muscle preparation and their antinociceptive activity was determined *in vivo* in PBQ induced writhing models (peripheral activity) and in the hot plate test (central activity) in rodents. Antagonism of antinociception by the peripheral opioid antagonist N-methylnalorphine and by comparison of the activities in the writhing and hot-plate tests demonstrated that the analgesic effects were predominantly mediated in the periphery. Peripheral analgesia was shown by a high potency in the writhing test coupled with a low potency in the hot plate test.

PBQ (phenyl-ρ-benzoquinone) induced writhing in mice is an assessment of both central and peripheral analgesia. For experimental protocol see Sigmund et al., Proc. Soc. Exp. Biol. Med., 95, p. 729 (1957) which is incorporated herein by reference. Central analgesia was determined by the inhibition of a hot plate response in mice. For experimental protocol see G. Woolfe and A. Macdonald, J. Pharmacol. Exp. Ther., 80, p.300 (1944) which is incorporated herein by reference. Assays measuring opioid receptor binding affinities for µ and δ receptors as well as GPI and MVD assays were determined through experimental protocol set out in Schiller et al., Biophys. Res. Commun., 85, p.1322 (1975) incorporated herein by reference.

The compounds of the present invention were prepared using solid phase synthesis as outlined below and generally known to persons skilled in the art.

### EXAMPLE 1

### Solid Phase Peptide Syntheses of Opioid Peptides

The synthetic peptides were prepared using Rink*¹ resin, 4-(2', 4'-Dimethoxy-phenyl-Fmoc-aminomethyl)-phenoxy Resin (Novabiochem or Advanced Chemtech) and the relevant C-terminal Nα-Fmoc-L-Amino acid residue of each peptide to be synthesized.
* denotes trade-mark

All L- and D-amino acids (Novabiochem of Advanced Chemtech) had their alpha group Fmoc-protected (9-fluorenyl-methyloxycarbonyl) and the following side chain protection groups: t-butyl ether (tBu) for serine, threonine and tyrosine; t-butyl ester (OtBu) for aspartic acid and glutamic; t-butyloxycarbonyl (tBoc) for lysine and 2,2,5,7,8-pentamethylchroman-6-sulphonyl (pmc) for arginine and trityl (trt) for cysteine.

Dimethylformamide (Anachemia) dimethylamine-free purity and was treated with activated 4 Å molecular sieves. Piperidine (Advanced Chemtech) was used without further purification. DCC (dicydohexylcarbodiimide) and HOBt (hydroxybenzotriazole) were obtained from Fluka and Advanced Chemtech respectively.

Solid phase peptide synthesis was carried out manually on Rink*² resin. Loading was approximately 0.6 mmole/g. Peptide condensation was carried out using: 1) Coupling: 2 equivalents each of Fmoc-amino acid, HOBt and DCC in DMF for 1-4 hours at room temperature. 2) Recoupling: 1 equivalent each of Fmoc-amino acid, HOBt and DCC. 3) Acetylation: 20% (v/v) (CH₃CO)₂O/DCM for 1 hour at room temperature. 4) N-α-Fmoc deprotection: 20% (v/v) piperidine in DMF for 25 minutes.
* denotes trade-mark

The removal of side chain protecting groups (tBu, Boc, Trt, Pmc) and deavage of peptide from the resin were effected by TFA containing cocktail (v/v) 55/5/40 TFA/Anisole/DCM for 90 minutes at room temperature under N₂. The peptide was precipitated from diethyl ether, filtered and dried. The crude peptide was purified and analyzed by HPLC on reverse phase column with a gradient elution using 0.06% TFA/H₂O and 0.06% TFA/acetonitrile.

### EXAMPLE 2

### Hot Plate Assay

### Measurement of Analgesic Activity

For this test, CD #1 male mice weighing between 20 and 25g were used. The mice were weighed, marked, and divided into groups of 10.

The mice were usually treated by subcutaneous injection of the compound (or the standard or the medium) in an injection volume equivalent to 0.1 ml/10g p.c. (10ml/kg). If an antagonist such as Nalaxone or N-methyl-Levallorphan was used, it was administered intra-peritoneally 20 minutes before the compound (or the standard, or the medium) was administered. The injection volume was also 0.1 ml/10g p.c. The dose of the antagonist was 10 mg/kg.

The mice were individually evaluated for reaction time on the hot plate. The temperature of the hot plate (Sorel, model DS37) was set at 55°C. The mouse was observed for signs of discomfort such as licking or shaking of the paws, attempting to escape (jumping off the plate) or trembling. The reaction time was counted when one of these signs appears and was noted in "seconds". Each mouse was observed for a maximum period of 30 seconds so as to prevent damage to the paw tissue. The mice may be observed at different time intervals after administration of the compound (or medium, or standard). The time intervals may be 30, 60 or 120 minutes (or other).

For each time reading, the average reaction time of the control group was multiplied by 1.5. The reaction time of each treated mouse was compared to the "control average X 1.5.". If the reaction time was inferior to the "control average X 1.5.", the mouse was considered to not have had an analgesic effect. If the reaction time was superior to the "control average X 1.5" then the mouse was considered to have had an analgesic effect. The number of analgesic mice in a group determined the analgesic percentage of the compound for this reading. If the analgesic percentage was inferior to 30%, the compound was considered inactive.

### EXAMPLE 3

### Writhing Assay

### Measurement of Contortions

The test was performed on CD #1 male mice weighing between 18 and 22g. The mice were weighed and marked. They were injected, by intra-peritoneal route, with 0.3ml/20g by weight with a solution of phenylquinone at 0.02%. The contortions which appeared during a 15 minute time period following the injection were counted. The phenylquinone was injected at time intervals of 5, 20 or 60 minutes after administration of the compound (or medium, or standard) by subcutaneous route. It was injected at time intervals of 60 minutes after the administration of the compound (or medium, or standard) by oral route;

The 0.02% phenylquinone³ solution was prepared in the following fashion. 20mg of phenylquinone was dissolved in 5ml ethanol 90% (sigma, reagent, alcohol). The dissolved phenylquinone was slowly added to 95 ml of distilled water continuously shaken and preheated (not boiled). The phenylquinone solution was, at all times protected from light and a new solution was prepared every day for the test. It is recommended to wait 2 hours before using the phenylquinone solution.
** 2-phenyl-1,4-benzoquinone (Sigma)

The test may be carried out on 5 mice at the same time. Each group usually contained 10 mice. If an antagonist, such as naloxone, was used, it was administered 20 minutes before the compound (or the medium, or the standard) by intra-peritoneal route.

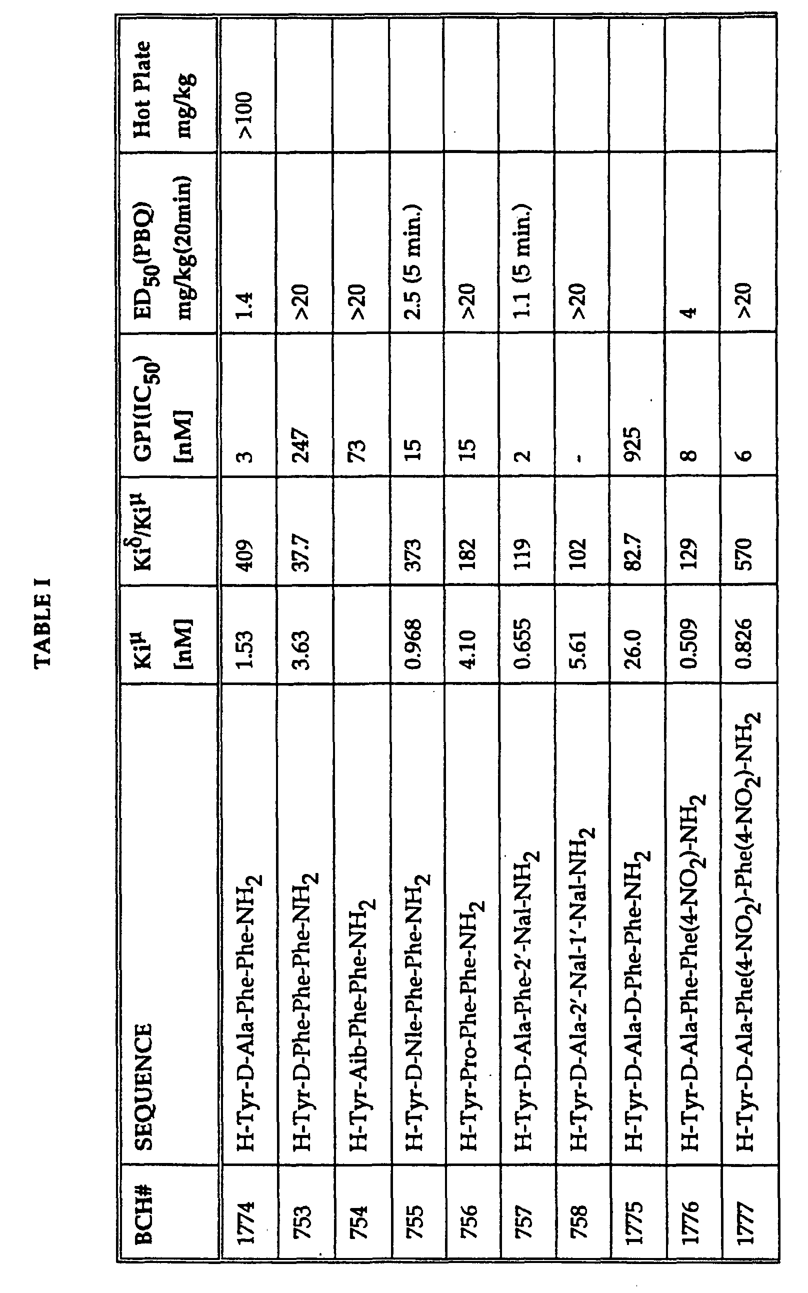

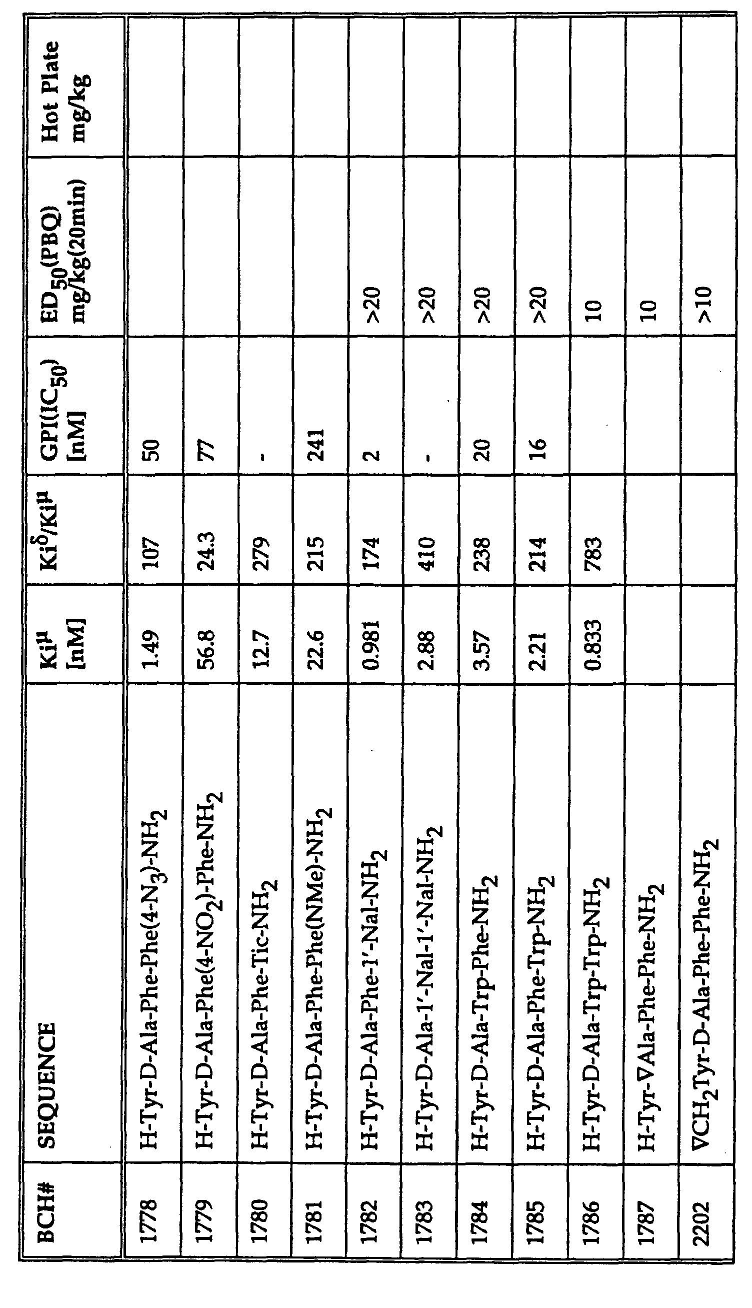

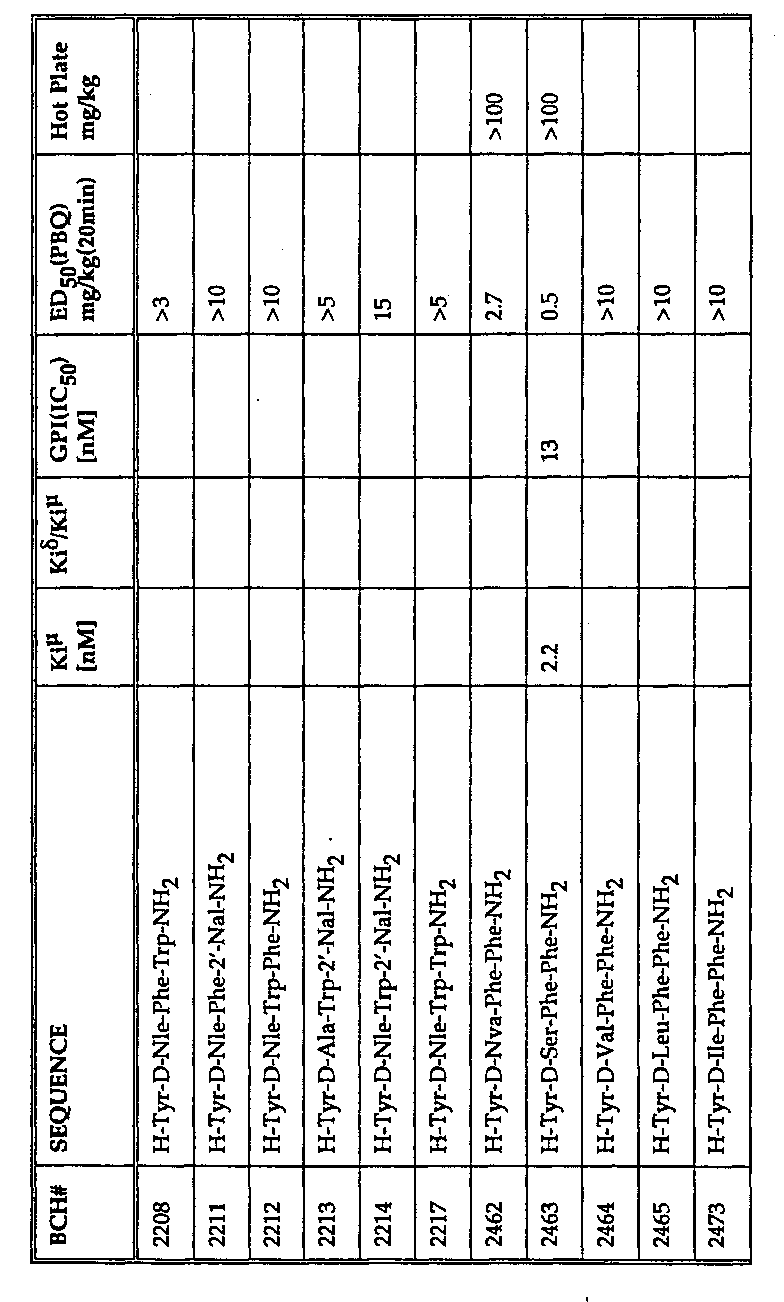

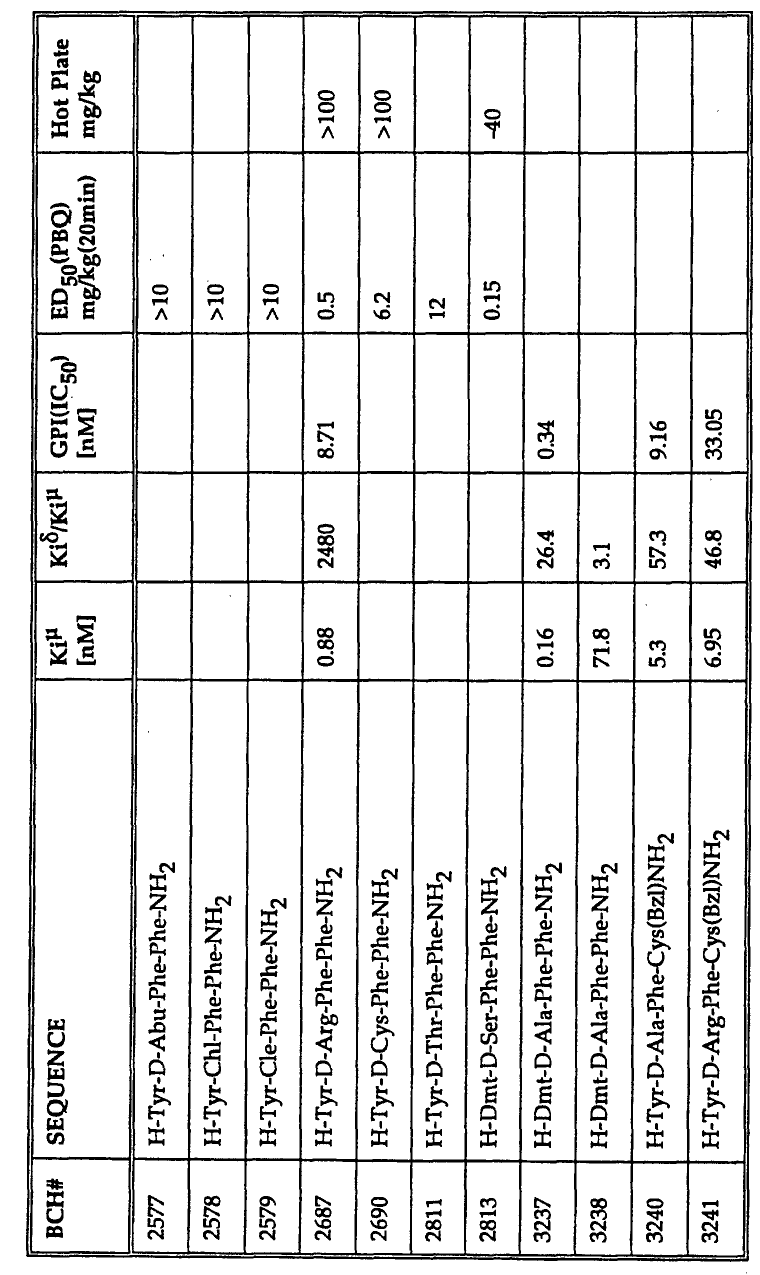

## Claims

1. A compound of the formula (1): 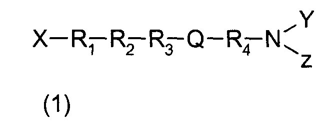 and derivatives and analogues thereof with peripheral analgesic effect,
wherein
X is selected from the group consisting of H and C₁₋₆ alkyl;
Y and Z are independently selected from the group consisting of H, cyclic aralkyl, and C₁₋₆ alkyl;
R₁ is a tyrosyl residue, 2', 6'-dimethyltyrosyl residue, or an analogue or derivative thereof;
R₃ is an aromatic amino acid;
R₄ is an aromatic amino acid residue;
R₂ is an amino acid having the R-configuration with the proviso that
when R₁ is a tyrosyl residue, R₂ is D-alanine, X, Y, and Z are H, and R₃ is phenylalanine, then R₄ is not phenylalanine unsubstituted or substituted with 4NO₂ or 4N₃;
when R₁ is a tyrosyl residue, R₂ is D-alanine, X, Y, and Z are H, and R₄ is phenylalanine, then R₃ is not phenylalanine unsubstituted or substituted with 4NO₂; when R₁ is a tyrosyl residue, R₂ is D-alanine, X, Y, and Z are H, and R₄ is 1'-naphthylalanine, then R₃ is not 1'-naphthylalanine or 2'-naphthylalanine; and
when R₁ is a tyrosyl residue, R₂ is D-alanine, and X, Y and Z are H, then both R₃ and R₄ are not tryptophan; and
Q is an amide bond or an interposed amide bond mimetic and whereby
H-Tyr-D-Phe-Phe-Phe-NH₂,
H-Tyr-D-NMePhe-Phe-Phe-NH₂,
H-Tyr-D-Tic-Phe-Phe-NH₂,
H-Tyr-Pro-Phe-Thr(Bzl)-NH₂,
H-Tyr-Pro-Phe-Phe-NH₂,
H-Tyr-Pro-Phe-Apb-NH₂,
H-Tyr-Pro-Phe-App-NH₂,
H-Tyr-Pro-Phe-Aph-NH₂,
H-Tyr-Pro-Apb-Phe-NH₂
wherein Apb means 2-amino-4-phenylbutanoic acid, App 2-amino-5-phenylpentanoic acid and Aph 2-amino-6-phenylhexanoic acid are excluded.

2. A compound of formula (1) according to claim 1,
wherein X is H.

3. A compound of formula (1) according to claim 1 or 2,
wherein
R₂ is an amino acid residue having the R-configuration with the proviso that where R₁ is a tyrosyl residue, R₂ is D-alanine, and Y and Z are H, then R₃ and R₄ are different and are selected from the group consisting of phenylalanine, and tryptophan.

4. A compound of formula (1) according to claim 1 or claim 2,
wherein
Q is an amide bond or an interposed amide bond mimetic of the formula Q₁-Q₂ wherein Q₁ is selected from the group consisting of CH₂, CHOH, C=O, C=S, and CH=, and Q₂ is selected from the group consisting of CH₂, NH, S, SO, SO₂, O, and CH=, with the proviso that when Q₁ is CH=, then Q₂ is CH=.

5. A compound of formula (1) according to claim 3,
wherein
Q is an amide bond or an interposed amide bond mimetic of the formula Q₁-Q₂ wherein Q₁ is selected from the group consisting of CH₂, CHOH, C=O, C=S, and CH=, and Q₂ is selected from the group consisting of CH₂, NH, S, SO, SO₂, O, and CH=, with the proviso that when Q₁ is CH=, then Q₂ is CH=.

6. A compound of formula (1) according to claim 1, 2 or 5,
wherein
Y and Z are H;
R₃ and R₄ are independently an aromatic amino acid; and
R₂ is an amino acid having the R-configuration with the proviso that when R₁ is a tyrosyl residue, R₂ is D-alanine, then R₃ and R₄ are different and are selected from the group consisting of phenylalanine and tryptophan.

7. A compound of formula (1) according to claim 4,
wherein
Y and Z are H;
R₃ and R₄ are independently an aromatic amino acid; and
R₂ is an amino acid having the R-configuration with the proviso that when R₁ is a tyrosyl residue, R₂ is D-alanine, then R₃ and R₄ are different and are selected from the group consisting of phenylalanine and tryptophan.

8. A compound of formula (1) according to claim 6,
wherein
R₂ is an amino acid having the R-configuration with the proviso that R₂ is not D-alanine; and
R₃ and R₄ are phenylalanyl residues.

9. A compound of formula (1) according to claim 7,
wherein
R₂ is an amino acid having the R-configuration with the proviso that R₂ is not D-alanine; and
R₃ and R₄ are phenylalanyl residues.

10. A compound according to claim 6, wherein
R₁ is a tyrosyl residue;
R₂ is selected from the group consisting of D-norvaline, D-serine and D-arginine;
R₃ and R₄ are phenylalanyl residues; and
Q is an amide bond.

11. A compound according to claim 7,
wherein
R₁ is a tyrosyl residue;
R₂ is selected from the group consisting of D-norvaline, D-serine and D-arginine;
R₃ and R₄ are phenylalanyl residues; and
Q is an amide bond.

12. A compound according to claim 1 selected from the group consisting of:
H-Tyr-D-Phe-Phe-Phe-NH₂;
H-Tyr-Aib-Phe-Phe-NH₂;
H-Tyr-D-Nle-Phe-Phe-NH₂;
H-Tyr-D-Ala-Phe-2'-Nal-NH₂;
H-Tyr-D-Ala-D-Phe-Phe-NH₂;
H-Tyr-D-Ala-Phe(4NO₂)-Phe(4NO₂)-NH₂;
H-Tyr-D-Ala-Phe-Tic-NH₂;
H-Tyr-D-Ala-Phe-Phe(NMe)-NH₂;
H-Tyr-D-Ala-Phe- 1'Nal-NH₂;
H-Tyr-D-Ala-Trp-Phe-NH₂;
H-Tyr-D-Ala-Phe-Trp-NH₂;
H-Tyr-∇Ala-Phe-Phe-NH₂;
∇CH₂-Tyr-D-Ala-Phe-Phe-NH₂;
H-Tyr-D-Nle-Phe-Trp-NH₂;
H-Tyr-D-Nle-Phe-2'-Nal-NH₂;
H-Tyr-D-Nle-Trp-Phe-NH₂;
H-Tyr-D-Ala-Trp-2'-Nal-NH₂;
H-Tyr-D-Nle-Trp-2'-Nal-NH₂;
H-Tyr-D-Nle-Trp-Trp-NH₂;
H-Tyr-D-Nva-Phe-Phe-NH₂;
H-Tyr-D-Ser-Phe-Phe-NH₂;
H-Tyr-D-Val-Phe-Phe-NH₂;
H-Tyr-D-Leu-Phe-Phe-NH₂;
H-Tyr-D-Ile-Phe-Phe-NH₂;
H-Tyr-D-Abu-Phe-Phe-NH₂;
H-Tyr-Chl-Phe-Phe-NH₂;
H-Tyr-Cle-Phe-Phe-NH₂;
H-Tyr-D-Arg-Phe-Phe-NH₂;
H-Tyr-D-Cys-Phe-Phe-NH₂;
H-Tyr-D-Thr-Phe-Phe-NH₂;
H-DMT-D-Ser-Phe-Phe-NH₂;
Tyr-D-Ala-Phe-Phe-OH trifluoroacetate;
H-Tyr-D-Ala-Phe-Phg-NH₂ trifluoroacetic acid salt;
H-Tyr-D-Arg-Phe-Hph-NH₂ bis-trifluoroacetic acid;
H-DMT-D-Ala-Phe-Phe-NH₂ trifluoroacetic acid;
H-D-DMT-D-Ala-Phe-Phe-NH₂ trifluoroacetic acid salt;
H-Tyr-D-Ala-Phe-Hph-NH₂ trifluoroacetic acid salt;
H-Tyr-D-Ala-Phe-Cys(Bzl)-NH₂ trifluoroacetic acid salt;
H-Tyr-D-Arg-Hph-Phe-NH₂ bis-trifluoroacetic acid salt;
H-Tyr-D-Arg-Phg-Phe-NH₂ bis-trifluoro acetic acid salt;
Tyr-D-Ala-Phe-Phe-CH₂OH hydrochloride salt;
H-Tyr-D-Ala-Hph-Phe-NH₂ trifluoroacetic acid salt;
H-Tyr-D-Met-Phe-Phe-NH₂ trifluoroacetic acid salt;
H-Tyr-D-Arg-Phe-D-Phe-NH₂ bis-trifluoroacetic acid salt;
H-Tyr-D-Ala-Phg-Phe-NH₂ trifluoroacetic acid salt;
H-Tyr-(D)-Ala-(D)-Phg-Phe-NH₂ trifluoroacetic acid salt;
H-Tyr-D-Arg-Phe-Phe(pf)-NH₂ bis-trifluoroacetic acid salt;
H-Tyr-D-Arg-Phe-D-Phe(pf)-NH₂ ditrifluoroacetic acid salt;
H-Tyr-D-Ala-Phe-Phe(pf)-NH₂ trifluoroacetic acid salt;
H-Tyr-D-Ala-Phe-D-Phe(pf)-NH₂ trifluoroacetic acid salt;

13. A compound according to claim 1 wherein said compound is H-Tyr-D-Nva-Phe-Phe-NH₂.

14. A compound according to claim 1 wherein said compound is H-Tyr-D-Ser-Phe-Phe-NH₂.

15. A compound according to claim 1 wherein said compound is H-Tyr-D-Arg-Phe-Phe-NH₂.

16. A pharmaceutical composition possessing analgesic activity, comprising an effective amount of at least one compound according to claim 1, 12, 13, 14 or 15, in admixture with a pharmaceutically acceptable carrier

17. A pharmaceutical composition possessing peripheral analgesic activity, comprising an effective amount of at least one compound according to claim 3 in admixture with a pharmaceutically acceptable carrier.

18. A pharmaceutical composition possessing peripheral analgesic activity, comprising an effective amount of at least one compound according to claim 4 in admixture with a pharmaceutically acceptable carrier.

19. A pharmaceutical composition possessing peripheral analgesic activity, comprising an effective amount of at least one compound according to claim 6 in admixture with a pharmaceutically acceptable carrier.

20. A pharmaceutical composition according to claim 16, further comprising an effective amount of at least one other therapeutically active agent.

21. A pharmaceutical composition according to claim 17, 18 or 19 further comprising an effective amount of at least one other therapeutically active agent.

22. The use of a compound H-Tyr-D-Ala-Phe-Phe-NH₂ or analogues or pharmaceutical derivatives thereof, for the manufacture of a peripheral analgesic for the treatment of pain.

23. The use of a compound according to claim 1, 12, 13, 14 or 15, and pharmaceutical derivatives thereof, for the manufacture of a peripheral analgesic for the treatment of pain.

24. The use of a compound according to claim 3 and pharmaceutical derivatives thereof, for the manufacture of a peripheral analgesic for the treatment of pain.

25. The use of a compound according to claim 4 and pharmaceutical derivatives thereof, for the manufacture of a peripheral analgesic for the treatment of pain.

26. The use of a compound according to claim 6 and pharmaceutical derivatives thereof, for the manufacture of a peripheral analgesic for the treatment of pain.

27. The use accorfding to claim 22, wherein said analogue is selected from:
H-Tyr-D-Ala-Phe-Phe (4-NO₂)-NH₂, and
H-Tyr-D-Ala-Phe-Phe- (4-N₃)-NH₂.
